# EUROPEAN PATENT APPLICATION

(11) **EP 2 399 589 A1**
(43) Date of publication of application: **28.12.2011**
(21) Application number: 09840407.2
(22) Date of filing: 18.09.2009
(51) Int. Cl.: A61K 31/715, A61K 31/717, A61L 31/00, A61P 25/00, A61P 25/02, A61P 41/00, C08B 15/05

(54) **HYDROGEL OF POLYSACCHARIDE DERIVATIVES**

(30) Priority: 19.02.2009 JP 2009036534
(71) Applicant: Teijin Limited, Osaka-shi, Osaka 541-0054 (JP); National University Corporation Nagoya University, Aichi 464-8601 (JP)
(72) Inventor: ENDO, Nobuyuki, Hino-shi Tokyo 191-0065 (JP); ITO, Masaya, Hino-shi Tokyo 191-0065 (JP); KANEKO, Hiroaki, Hino-shi Tokyo 191-0065 (JP); HIRATA, Hitoshi, Nagoya-shi Aichi 464-8601 (JP); YAMAMOTO, Michiro, Nagoya-shi Aichi 464-8601 (JP)
(74) Representative: Hallybone, Huw George
(86) International application number: PCT/JP2009/066858
(87) International publication number: WO 2010/095304

(57) **Abstract**

A nerve dysfunction repairing material including a hydrogel of a polysaccharide derivative that has, in a 0.5 wt% aqueous solution, a complex modulus of 1 to 1000 N/m² and a loss factor of 0.01 to 2.0 as measured at an angular velocity of 10 rad/sec using a dynamic viscoelasticity measuring apparatus. The nerve dysfunction repairing material can be a hydrogel injectable through a syringe, has excellent retention in the body, and has a restorative effect on the function of damaged or degenerated nerves.

## Description

### Technical Field

The present invention relates to an injectable nerve dysfunction repairing material including a polysaccharide derivative.

### Background Art

An accident, overuse of the body, or the like causes nerve damage and/or degeneration, impairing its function. In particular, examples of diseases accompanied by nerve dysfunction related to peripheral nerves include nerve damage, carpal tunnel syndrome, and cubital tunnel syndrome. For such a nerve with impaired function, it takes a long period of time to restore its function, and the burden on the patient is great.

Nowadays, decompression such as neurolysis has been performed against carpal tunnel syndrome, cubital tunnel syndrome, and like entrapment syndromes, and it has been reported effective. However, there are some problems concerned, including postoperative tissue scarring around the nerve, fibrosis in a bundle of nerve fibers, etc. For example, although the failure rate in carpal tunnel release is 3%, the main reason for the recurrence of symptom is believed to be postoperative adhesion or fibrosis inside or outside the nerve, and it is expected that when postoperative adhesion is reduced, the effectiveness of the repair of nerve dysfunction will increase.

Meanwhile, to deal with such problems, a bioabsorbable anti-adhesion material has been proposed and approved as ADCON (Gliatech) in the U.S., and was in clinical use. However, regarding this anti-adhesion material, there has been a concern about side effects due to the delay of healing of the surgery site. That is, even if postoperative adhesion can be prevented, the repair of nerve dysfunction after decompression may be hindered by the delay of healing, etc.

In the past, in order to restore impaired nerve function, various proposals using a polysaccharide, a biocompatible material, have been made. For example, a therapeutic material for neurological disorders has been disclosed, which has a lipid-bound glycosaminoglycan or a salt thereof as an active ingredient (JP-A-9-30979). Although this therapeutic material for neurological disorders can be formulated in any dosage form, there is no description about a gel having moderate viscosity related to retention in the body. Further, there is no description or suggestion about postoperative adhesion.

Further, a material for nerve regeneration has been disclosed, which includes a cross-linkable polysaccharide obtained by covalent cross-linking of a carboxyl-group-containing polysaccharide and/or a salt thereof using a cross-linking reagent including an amine-based compound (JP-A-2000-198738). However, concerns still remain about the safety, for example, inflammatory reaction by the residual cross-linking reagent. Further, a chemically cross-linked gel may undergo property changes due to heterogeneity, and there is room for improvement in stability. In addition, there is no description or suggestion about postoperative adhesion.

Further, it has been reported that, using a rabbit sciatic nerve adhesion model, hyaluronic acid inhibited the postoperative adhesion of the peripheral nerves and also inhibited the delay of peripheral nerve latency (The British Association of Plastic Surgeons 56, pp 342-347, 2003). However, such hyaluronic acid is not effective unless applied from the start of surgery, and this interferes with surgery in the actual use, so there is room for improvement in the handleability.

Meanwhile, regarding a gel material using cellulose as a polysaccharide, WO2007/015579 discloses, in the Description, a derivative obtained by modifying carboxymethylcellulose with phosphatidylethanolamine, which is dissolved in water to form a gel. However, there is no description or suggestion about a nerve dysfunction repairing effect.

As a material for preventing the postoperative adhesion of the peripheral nerves, HYALOGLIDE® (Fidia Advanced Biopolymers), an auto-cross-linked hyaluronic acid that has already been in clinical use in Europe, has been reported effective in the alleviation of hand pain after surgery of the peripheral nerves or Tinel's sign (Microsurgery 27 (1), pp 2-7, 2007).

As described above, although various proposals have been made for the restoration of nerve function using a polysaccharide, no study has been made on a hydrogel using a phospholipid-modified polysaccharide derivative, which has a nerve dysfunction repairing effect, has moderate viscosity, causes less postoperative adhesion, and is easy to handle. In particular, a polysaccharide derivative that shows a nerve conduction velocity improving effect is heretofore unknown.

### Disclosure of the Invention

An object of the inveniton is to provide a nerve dysfunction repairing material for restoring the function of damaged or degenerated nerves, and in particular to provide a nerve dysfunction repairing material that can be a hydrogel injectable through a syringe and has excellent retention in the body.

The present inventors conducted extensive research on nerve dysfunction repairing materials for restoring the function of damaged or degenerated nerves. As a result, they found the presence of a hydrogel of a polysaccharide derivative characterized by having, in a 0.5 wt% aqueous solution, a complex modulus of 1 to 1000 N/m² and a loss factor of 0.01 to 2.0 as measured at an angular velocity of 10 rad/sec using a dynamic viscoelasticity measuring apparatus. They also found that such a hydrogel is useful in the restoration of the function of damaged nerves and causes less postoperative adhesion, and thus accomplished the invention.

Specifically, the invention is a nerve dysfunction repairing material including a hydrogel of a polysaccharide derivative that has, in a 0.5 wt% aqueous solution, a complex modulus of 1 to 1000 N/m² and a loss factor of 0.01 to 2.0 as measured at an angular velocity of 10 rad/sec using a dynamic viscoelasticity measuring apparatus.

When the polysaccharide derivative used in the invention is dissolved in water, it turns into a hydrogel having a specific modulus and viscosity to allow injection. When such a hydrogel is used as an injectable gel for medical use, it has effects as a nerve dysfunction repairing material, for example, a nerve conduction velocity improving effect.

Further, the nerve dysfunction repairing material of the invention has moderate viscoelasticity and/or excellent retention in the body, and thus has excellent handleability and can be applied to regions of complex configurations at the time of surgery using an endoscope, etc.

### Brief Description of the Drawings

Fig. 1 shows the nerve conduction velocity improving effect of a nerve dysfunction repairing material of the invention in 20 days after surgery.
Fig. 2 shows the increase in the complex modulus of a nerve dysfunction repairing material of the invention at a physiological salt concentration.
Fig. 3 shows perineurium regeneration by a hydrogel of a polysaccharide derivative of the invention in one week after surgery. The arrow shows the perineurium.
Fig. 4 shows perineurium regeneration in one week after surgery in the case of not using a hydrogel of a polysaccharide derivative of the invention. The arrow shows the perineurium.
Fig. 5 shows myelin sheath regeneration by a hydrogel of a polysaccharide derivative of the invention in 6 weeks after surgery. The arrow shows the myelin sheath.
Fig. 6 shows myelin sheath regeneration in 6 weeks after surgery in the case of not using a hydrogel of a polysaccharide derivative of the invention. The arrow shows the myelin sheath.

### Best Mode for Carrying Out the Invention

The invention is a nerve dysfunction repairing material including a hydrogel of a polysaccharide derivative that has, in a 0.5 wt% aqueous solution, a complex modulus of 1 to 1000 N/m² and a loss factor of 0.01 to 2.0 as measured at an angular velocity of 10 rad/sec using a dynamic viscoelasticity measuring apparatus.

The complex modulus range is preferably 1 to 200 N/m², and more preferably 1 to 100 N/m². Further, the loss factor is preferably 0.01 to 1.5.

The polysaccharide derivative used in the invention is preferably a cellulose derivative, and may more preferably be a cellulose derivative having a repeating unit represented by the following formula: 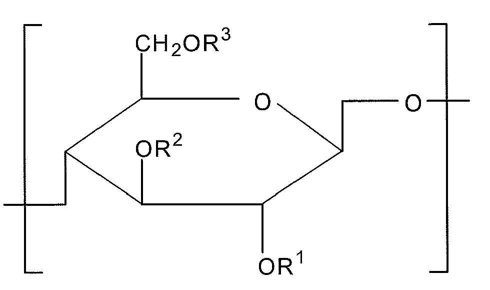 wherein R¹, R², and R³ are each independently selected from the group consisting of the following formulae (a), (b), (c), and (d) :

-H (a) ;

-CH₂-COOH (b) ;

-CH₂-COOX (c) ;

and 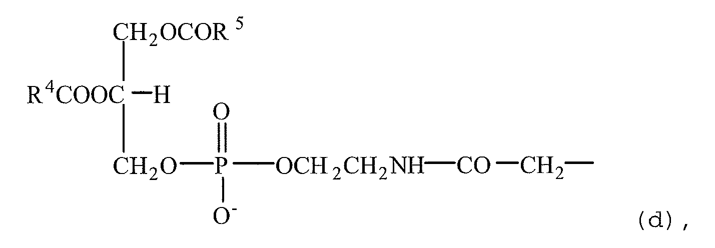 wherein
X in the formula (c) is an alkali metal or an alkaline-earth metal,
R⁴ and R⁵ in the formula (d) are each independently a C₉₋₂₇ alkyl group or alkenyl group,
the total degree of substitution of (b) and (c) is 0.3 to 2.0, and
the degree of substitution of (d) is 0.001 to 0.05, and more preferably 0.005 to 0.015.

In the above formula, R⁴ and R⁵ are each independently a C₉₋₂₇ alkyl group or alkenyl group. In particular, it is preferable that R⁴ and R⁵ are C₉₋₁₉ alkenyl groups. Among them, it is preferable that R⁴CO- and/or R⁵CO- is an oleoyl group, and it is particularly preferable that R⁴CO- and R⁵CO- are oleoyl groups.

The nerve dysfunction repairing material of the invention is preferably a nerve dysfunction repairing material that is an injectable hydrogel containing 0.1 to 1.5 parts by weight of the polysaccharide derivative used in the invention per 100 parts by weight of water. It is still more preferably 0.5 to 1.0 part by weight.

Among them, it is preferable that the complex modulus in a 0.5 wt% aqueous solution is 1 to 200 N/m² as measured at an angular velocity of 10 rad/sec using a dynamic viscoelasticity measuring apparatus. It is still more preferably 1 to 100 N/m². Further, it is preferable that the loss factor at this time is 0.01 to 1.5.

Further, it is preferable that the nerve dysfunction repairing material of the invention includes a hydrogel of a polysaccharide derivative, and that at a physiological salt concentration, the complex modulus in a 1.0 wt% aqueous solution increases by 1 to 1000 N/m² as measured at an angular velocity of 10 rad/sec using a dynamic viscoelasticity measuring apparatus. The range of viscoelasticity increase is more preferably an increase by 50 to 700 N/m², and still more preferably an increase by 100 to 500 N/m².

A physiological salt concentration herein means the salt concentration of a physiological salt solution adjusted to allow cell survival. As specific salt concentrations, physiologic saline (0.9% aqueous NaCl solution), Ringer's solution, phosphate buffer, and the like can be mentioned as examples.

The invention is a nerve dysfunction repairing material. For example, it is suitably used to restore the function of nerves damaged and/or degenerated due to an accident, overuse of the body, etc.

When a cellulose derivative is used as the polysaccharide derivative for the preparation of the nerve dysfunction repairing material of the invention, the production may be follows, for example.

### <Cellulose Derivative Production Method>

The cellulose derivative used in the invention mentioned above can be produced by a method including a step in which carboxymethylcellulose having a repeating unit represented by the following formula and a molecular weight of 5 × 10³ to 5 × 10⁶ : 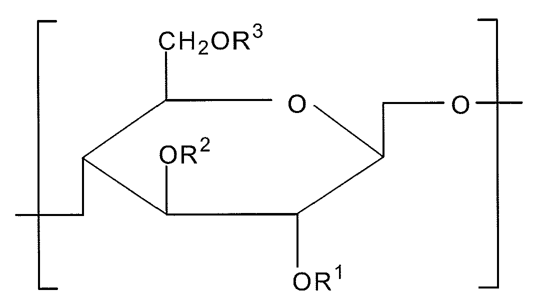 and phosphatidylethanolamine represented by the following formula: 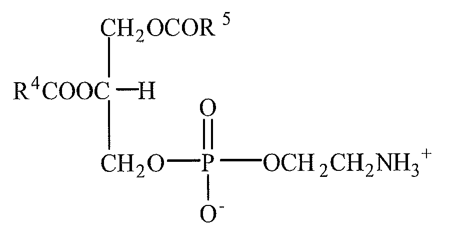 in such proportions that the amount of phosphatidylethanolamine is 0.1 to 100 equivalents per 100 equivalents of the carboxyl groups of carboxymethylcellulose (i.e., the total of the substituents of (b) + (c)) are dissolved in a mixed solvent including water and a water-compatible organic solvent and having the water in an amount of 20 to 70% by volume, and then allowed to react in the presence of a condensing agent.

R¹, R², and R³ herein are each independently selected from the following formulae (a), (b), and (c):

-H (a) ;

-CH₂-COOH (b) ;

and

-CH₂-COOX (c),

wherein
X in the formula (c) is an alkali metal or an alkaline-earth metal,
the total degree of substitution of the formulae (b) and (c) is 0.3 to 2.0, and
R⁴ and R⁵ are each independently a C₉₋₂₇ alkyl group or alkenyl group.

The carboxymethylcellulose as a raw material preferably has a molecular weight of 5 × 10³ to 5 × 10⁶, more preferably 5 × 10⁴ to 5 × 10⁶, and still more preferably to 5 × 10⁴ to 1 × 10⁶.

The carboxymethylcellulose as a raw material can be produced, for example, by dissolving pulp in a sodium hydroxide solution, and etherifying the same with monochloroacetic acid or a sodium salt thereof, followed by purification.

The alkali metal of X in the formula (c) is preferably sodium, potassium, lithium, or the like, and the alkaline-earth metal is preferably magnesium, calcium, or the like.

The total degree of substitution of (b) and (c) is 0.3 to 2.0, preferably 0.5 to 1.8, and more preferably 0.6 to 1.5. The proportions of (b) and (c) are not particularly limited. However, in terms of solubility in water, it is preferable that (c) is in excess of (b).

The specific structural formula of preferred carboxymethylcellulose as a raw material is as shown by the following formula. With respect to the substitution position of the carboxymethyl group in the cellulose skeleton, it is preferably at C-6.

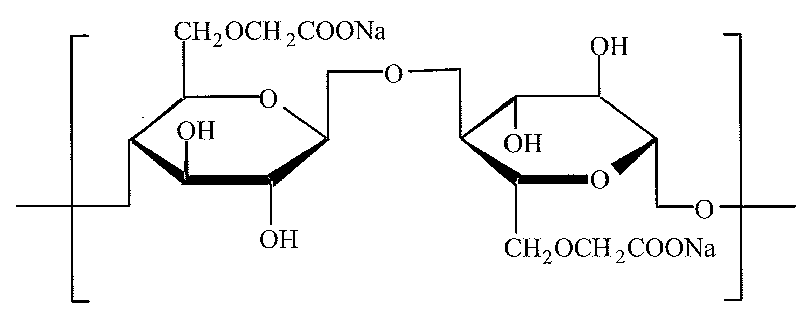

In phosphatidylethanolamine represented by the above formula for use in the cellulose derivative production method, R⁴ and R⁵ are each independently a C₉-₂₇ alkyl group or alkenyl group. It is preferable that R⁴ and R⁵ are each a C₉₋₂₇ alkenyl group. In particular, it is preferable that R⁴CO- and/or R⁵CO- is an oleoyl group, and it is particularly preferable that R⁴CO- and R⁵CO- are oleoyl groups.

The phosphatidylethanolamine as a raw material may be either extracted from animal tissue or synthetically produced. Specific examples thereof include dilauroylphosphatidylethanolamine, dimyristoylphosphatidylethanolamine, dipalmitoylphosphatidylethanolamine, distearoylphosphatidylethanolamine, diarachidoylphosphatidylethanolamine, dibehenoylphosphatidylethanolamine, lauroleoylphosphatidylethanolamine, myristoleoylphosphatidylethanolamine, palmitoleoylphosphatidylethanolamine, dioleoylphosphatidylethanolamine, dilinoleoylphosphatidylethanolamine, dilinolenoylphosphatidylethanolamine, diarachidonoylphosphatidylethanolamine, and didocosahexaenoylphosphatidylethanolamine. Among these, dioleoylphosphatidylethanolamine is preferable in terms of solubility in the organic solvent used for synthesis. Phosphatidylethanolamine is a safe substance of biological origin.

It is believed that in the cellulose derivative used in the invention, phosphatidylethanolamine enhances the hydrophobic interaction between cellulose derivative molecules, and, as a result, the cellulose derivative used in the invention forms a hydrogel.

Carboxymethylcellulose and phosphatidylethanolamine, which are raw materials of the cellulose derivative used in the invention, are allowed to react in such proportions that the amount of phosphatidylethanolamine is 0.1 to 50 equivalents, preferably 1 to 40 equivalents, more preferably 3 to 30 equivalents, per 100 equivalents of the carboxyl groups of carboxymethylcellulose. When the amount is less than 0.1 equivalents, the resulting cellulose derivative does not form a hydrogel. When the amount is more than 40 equivalents, no increase in viscoelasticity is observed at physiological salt concentrations.

In the condensation reaction between carboxymethylcellulose and phosphatidylethanolamine, the reaction efficiency may decrease depending on the reactivity of the condensing agent used for condensation or the reaction conditions. Therefore, it is preferable that phosphatidylethanolamine is used in excess of the calculated value of the desired degree of substitution.

Carboxymethylcellulose and phosphatidylethanolamine are dissolved in a mixed solvent including water and a water-compatible organic solvent (A), the water being present in an amount of 20 to 70% by volume. When the water content is less than 20% by volume, carboxymethylcellulose is less soluble, while when it is more than 70% by volume, phosphatidylethanolamine is less soluble, whereby the reaction does not proceed. The water content is preferably 30 to 60% by volume.

Specific examples of water-compatible organic solvents (A) include organic solvents having a cyclic ether bond, such as tetrahydrofuran, 1,4-dioxane, 1,3-dioxane, 1,3-dioxolane, and morpholine, organic solvents having an amide bond, such as dimethylacetamide, dimethylformamide, and N-methyl-2-pyrrolidone, amines such as pyridine, piperidine, and piperazine, and sulfoxides such as dimethyl sulfoxide. Among these, cyclic ethers and sulfoxides are preferable. In particular, tetrahydrofuran, dioxane, and dimethyl sulfoxide are more preferable.

As reagents used for the reaction, carboxyl activating agents and condensing agents are preferable. Examples of carboxyl activating agents include N-hydroxysuccinimide, p-nitrophenol, N-hydroxybenzotriazole, N-hydroxypiperidine, N-hydroxysuccinamide, 2,4,5-trichlorophenol, and N,N-dimethylaminopyridine. Examples of condensing agents include 4-(4,6-dimethoxy-1,3,5-triazin-2-yl)-4-methyl morpholinium chloride, 1-ethyl-3-(dimethylaminopropyl)-carbodiimide and the hydrochloride thereof, diisopropylcarbodiimide, dicyclohexylcarbodiimide, and N-hydroxy-5-norbornene-2,3-dicarboximide. Among these, it is preferable to use N-hydroxybenzotriazole as a carboxyl activating agent and 4-(4,6-dimethoxy-1,3,5-triazin-2-yl)-4-methyl morpholinium chloride or 1-ethyl-3-(dimethylaminopropyl)-carbodiimide hydrochloride as a condensing agent.

The reaction temperature is preferably 0°C to 60°C. In order to inhibit the production of by-products, the reaction is more preferably performed at 0 to 10°C. The reaction environment is preferably weakly acidic, and more preferably pH 6 to 7.

### <Cellulose Derivative Purification Method>

The cellulose derivative production method used in the invention may include, for the obtained cellulose derivative, a step of purifying the cellulose derivative using an organic solvent (B) that essentially does not dissolve carboxymethylcellulose but is compatible with water.

The organic solvent that essentially does not dissolve carboxymethylcellulose herein means such an organic solvent that, with respect to a carboxymethylcellulose sodium salt or carboxymethylcellulose (COOH type) available in powder or freeze-dried form, when the solubility of the carboxymethylcellulose in the organic solvent is examined in the absence of water, the solubility is 3% or less. Specific examples thereof include alcohols such as methanol, ethanol, n-propyl alcohol, isopropyl alcohol, n-butyl alcohol, and t-butyl alcohol, polyalcohols such as ethylene glycol, 1,2-propylene glycol, 1,3-propylene glycol, and glycerin, ketones such as acetone, and aromatic alcohols such as phenol. Among these, those having a boiling point of less than 100°C are preferable. For example, methanol, ethanol, and isopropyl alcohol are preferable. Considering the use in vivo, ethanol is particularly preferable.

When purification is performed using an organic solvent (B) from these groups, it is possible to employ a method in which the organic solvent (B) is added to a cellulose derivative contained in a mixture of water, the organic solvent (A), and the like to form a precipitate, thereby removing the cellulose derivative. Alternatively, it is also possible to employ a method in which the organic solvent (B) is added to the precipitate obtained above, a dry powder, or a sponge or like shaped body obtained by freeze-drying, thereby performing washing. By these purification methods, catalysts such as the condensing agent and carboxyl activating agent used for the reaction, unreacted phospholipid remaining unreacted in the system, and the like can be removed. In order to obtain the desired product suspended in the organic solvent (B), a method such as centrifugation or filtration is employed. Soxhlet extraction can also be employed for washing with the organic solvent (B).

### <Hydrogel of Cellulose Derivative>

The nerve dysfunction repairing material of the invention is a hydrogel containing the above-mentioned cellulose derivative. The hydrogel contains the cellulose derivative in an amount of 0.1 to 1.5 parts by weight, preferably 0.5 to 1.0 part by weight, per 100 parts by weight of water.

Such a hydrogel can be easily deformed when touched with a metal spatula, such as a spatula, and is in the state that allows easy application to the affected area. The hydrogel can also be injected with an instrument having a thin tube, such as a syringe.

The gel preferably has a complex modulus of 1 to 200 N/m², still more preferably 1 to 100 N/m², as measured at an angular velocity of 10 rad/sec using a dynamic viscoelasticity measuring apparatus under the condition where the polymer concentration in water is 0.5 wt% and the temperature is 37°C. Further, it is preferable that the loss factor at this time is 0.01 to 1.5. This is because this range is the most effective in the restoration of the function of damaged or degenerated nerves.

Further, the hydrogel of the invention is transparent and colorless. In industrial production, this is advantageous in that when foreign substances, such as dust, are incorporated in the process of production, such foreign substances can be detected.

Possible examples of components contained in the hydrogel other than water include condensing agents used as catalysts; by-products, such as urea, produced by a condensing agent undergoing a predetermined chemical reaction; carboxyl activating agents; unreacted phosphatidylethanolamines; foreign substances that may be incorporated in each stage of the reaction; and ions used for pH adjustment. However, these components are removed by purification or washing using the organic solvent (B) mentioned above, and it is preferable that the levels of all compounds are kept low so that their entry into the body is not recognized as a foreign-body reaction.

The method for storing the nerve dysfunction repairing material of the invention is not limited. For example, it can be stored in a cool, dark place, and brought back to room temperature before use and used. The method for sterilizing the nerve dysfunction repairing material of the invention is not limited either, and a method generally used for sterilizing medical instruments and medical materials may be employed, such as ethylene oxide gas sterilization, autoclave sterilization, gamma-ray sterilization, or electron beam sterilization.

Further, in the case where the nerve dysfunction repairing material of the invention is used after surgery, for example, about 0.1 to 5.0 mL is applied to the surgery site and the surrounding area with a syringe to cover the entire surgery area, whereby the restoration of the function of damaged or degenerated nerves can be expected.

### Examples

(1) The materials used in the Examples are as follows:
   (i) CMCNa: sodium carboxymethylcellulose (manufactured by Dai-ichi Kogyo Seiyaku, degree of substitution: 0.73; or manufactured by Nippon Paper Chemicals, degree of substitution: 0.69),
   (ii) tetrahydrofuran (manufactured by Wako Pure Chemical Industries),
   (iii) 0.1 M HCl (manufactured by Wako Pure Chemical Industries),
   (iv) 0.1 M NaOH (manufactured by Wako Pure Chemical Industries),
   (v) 4-(4,6-dimethoxy-1,3,5-triazin-2-yl)-4-methyl morpholinium chloride (manufactured by Kokusan Chemical),
   (vi) L-α-dioleoylphosphatidylethanolamine (COATSOME ME-8181, manufactured by NOF Corporation),
   (vii) ethanol (manufactured by Wako Pure Chemical Industries),
   (viii) distilled water for injection (manufactured by Otsuka Pharmaceutical),
   (ix) ethanol for disinfection (manufactured by Wako Pure Chemical Industries),
   (x) pentobarbital sodium (Nembutal injection, manufactured by Dainippon Sumitomo Pharma), and
   (xi) NaCl (manufactured by Wako Pure Chemical Industries).
(2) Measurement of Phospholipid Content in Cellulose Derivative
   The proportion of phospholipid in a cellulose derivative was determined from the analysis of the total phosphorus content by vanadomolybdate absorptiometry.
(3) Measurement of Complex Modulus and Loss Factor of Hydrogel
   The complex modulus and loss factor of a hydrogel were measured at 37°C and an angular velocity of 10 rad/sec using Rheometer RFIII (TA Instrument), a dynamic viscoelasticity measuring apparatus.
   Complex modulus refers to a constant that represents a ratio between the stress and strain of an elastic body. Loss factor refers to a constant that represents a ratio of between storage shear modulus and loss shear modulus.

### [Example 1]

### (Cellulose Derivative)

3000 mg of CMCNa (manufactured by Dai-ichi Kogyo Seiyaku, degree of substitution: 0.73) was dissolved in 600 mL of water, and 600 mL of tetrahydrofuran was further added thereto. To this solution were added 1405 mg (1.889 mol) of L-α-dioleoylphosphatidylethanolamine (20 equivalents per 100 equivalents of the carboxyl groups of CMCNa) and 575 mg (2.08 mol) of 4-(4,6-dimethoxy-1,3,5-triazin-2-yl)-4-methyl morpholinium chloride, followed by stirring overnight. After stirring, tetrahydrofuran was removed, water was evaporated to some extent, and the mixture was then added to ethanol to cause precipitation. Ethanol was removed by filtration, followed by washing with ethanol again. The residue was vacuum-dried to give a cellulose derivative, and the phospholipid content thereof was measured. On the assumption that the degree of substitution of sodium carboxymethylcellulose before the reaction was 0.73, and that all carboxymethyl groups were sodiated, the degree of substitution of the formula (d) was determined by calculation using the phospholipid content. The degree of substitution of the formula (d) was 0.78 mol%/sugar.

### (Hydrogel)

A composition made of the vacuum-dried cellulose derivative was sterilized and then dissolved in distilled water for injection to prepare a 0.5 wt% hydrogel. The complex modulus and loss factor of the obtained hydrogel were measured, and the results were 18.3 N/m² and 0.63, respectively.

### [Example 2]

### (Production of Rat Sciatic Nerve Dysfunction)

Using Lewis rats (three rats) from Charles River Laboratories Japan, sciatic nerve dysfunction was produced in accordance with the method of Ohsumi et al., [Hidehiko Ohsumi, Hitoshi Hirata, Takeshi Nagakura, Masaya Tsujii, Toshiko Sugimoto, Keiichi Miyamoto, Takeshi Horiuchi: Plastic and Reconstructive Surgery 116 (3) : 823-30, 2005]. That is, a rat was fixed in the lateral position under anesthesia with intraperitoneally administered pentobarbital sodium, and the gluteal region was shaved and then disinfected with ethanol for disinfection. From the abdominal region towards the dorsal region, a 4- to 5-cm incision was made in the gluteal region to expose the sciatic nerve. The epineurium and perineurium of the sciatic nerve were stripped off 1.5 cm, and further the surrounding muscle tissue was burned. Subsequently, the hydrogel of Example 1 (0.5 mL) was applied around the sciatic nerve having the epineurium and perineurium stripped off, and the muscle layer and skin at the incision site were sutured. The wound site was disinfected with an Isodine disinfectant, and the rat was then returned to the cage. In 20 days after surgery, the animals were subjected again to sciatic nerve exposure under pentobarbital sodium anesthesia, and nerve conduction velocity was measured using NeuroPack (Nihon Kohden). Significant differences were tested using Student's t-test. As a result, the nerve conduction velocity in 20 days after surgery was 18.8 ± 3.3 m/s (average ± standard deviation) in each case.

### [Comparative Example 1]

As control, the same operation as in Example 2 was performed without applying the hydrogel, and nerve conduction velocity was measured. As a result, the nerve conduction velocity was 11.8 ± 3.6 m/s (average ± standard deviation).

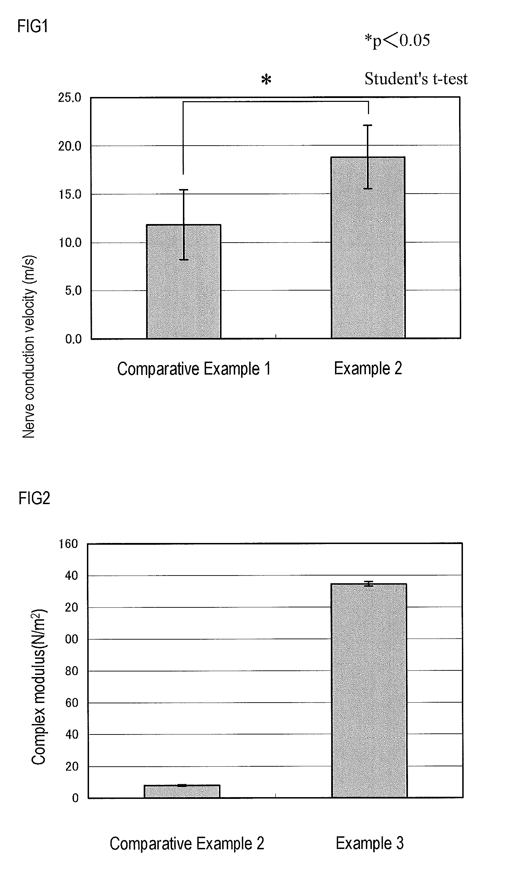

The results of the measurement of nerve conduction velocity in 20 days after surgery in Example 2 and Comparative Example 1 are shown in Fig. 1.

As above, the nerve conduction velocity in 20 days after surgery was statistically significantly greater in Example 2 than in Comparative Example 1. Therefore, it was confirmed that the hydrogel obtained in Example 1 is highly effective in restoring the function of damaged or degenerated nerves in vivo.

### [Example 3]

### (Increase in Complex Modulus by Addition of Salt)

3500 mg of CMCNa (manufactured by Nippon Paper Chemicals, degree of substitution: 0.69) was dissolved in 100 mL of water, and 100 mL of tetrahydrofuran was further added thereto. To this solution were added 413.7 mg (0.0000795 mol) of L-α-dioleoylphosphatidylethanolamine (5 equivalents per 100 equivalents of the carboxyl groups of CMCNa) and 169.4 mg (0.0000874 mol) of 4-(4,6-dimethoxy-1,3,5-triazin-2-yl)-4-methyl morpholinium chloride as a condensing agent, followed by stirring overnight. After stirring, the mixture was added to ethanol to cause precipitation. Then, the same operation as in Example 1 was performed to obtain a cellulose derivative. The degree of substitution was 1.0 mol%/sugar. 20 mg of a composition made of the cellulose derivative was dissolved in 1800 mg of distilled water for injection, and then 200 mg of 9% NaCl was added thereto to give a final concentration of 0.9%. A hydrogel with a final concentration of 1.0 wt% was thus prepared. The complex modulus of the obtained hydrogel was measured. The result was 134.5 ± 1.4 N/m² (average ± standard deviation).

### [Comparative Example 2]

A hydrogel was prepared by the same operation as in Example 3, except that 200 mg of distilled water for injection was added in place of 9% of NaCl . The complex modulus of the obtained hydrogel was measured. The result was 8.0 ± 0.5 N/m² (average ± standard deviation).

The results of complex modulus in Example 3 and Comparative Example 2 are shown in Fig. 2.

As above, the increase in complex modulus is greater in Example 3 than in Comparative Example 2, and it was confirmed that the complex modulus of a hydrogel having a complex modulus as low as 5 to 200 N/m² remarkably increases when NaCl is added thereto to give a concentration of 0.9 wt%, which is the same level as in vivo.

### [Example 4]

### (Cellulose Derivative)

3000 mg of CMCNa (manufactured by Dai-ichi Kogyo Seiyaku, degree of substitution: 0.73) was dissolved in 600 mL of water, and 600 mL of tetrahydrofuran was further added thereto. To this solution were added 1405 mg (1.889 mol) of L-α-dioleoylphosphatidylethanolamine (20 equivalents per 100 equivalents of the carboxyl groups of CMCNa) and 575 mg (2.08 mol) of 4-(4,6-dimethoxy-1,3,5-triazin-2-yl)-4-methyl morpholinium chloride, followed by stirring overnight. After stirring, tetrahydrofuran was removed, water was evaporated to some extent, and the mixture was then added to ethanol to cause precipitation. Ethanol was removed by filtration, followed by washing with ethanol again. The residue was vacuum-dried to give a cellulose derivative, and the phospholipid content thereof was measured. On the assumption that the degree of substitution of sodium carboxymethylcellulose before the reaction was 0.73, and that all carboxymethyl groups were sodiated, the degree of substitution of the formula (d) was determined by calculation using the phospholipid content. The degree of substitution of the formula (d) was 0.78 mol%/sugar.

### (Hydrogel)

A composition made of the vacuum-dried cellulose derivative was sterilized and then dissolved in distilled water for injection to prepare a 0.5 wt% hydrogel. The complex modulus and loss factor of the obtained hydrogel were measured, and the results were 18.3 N/m² and 0.63, respectively.

### [Example 5]

### (Production of Rat Sciatic Nerve Degeneration)

Using Lewis rats (three rats) from Charles River Laboratories Japan, the sciatic nerve was degenerated in accordance with the method of Ohsumi et al., [Hidehiko Ohsumi, Hitoshi Hirata, Takeshi Nagakura, Masaya Tsujii, Toshiko Sugimoto, Keiichi Miyamoto, Takeshi Horiuchi: Plastic and Reconstructive Surgery 116 (3) : 823-30, 2005] . That is, a rat was fixed in the lateral position under anesthesia with intraperitoneally administered pentobarbital sodium, and the gluteal region was shaved and then disinfected with ethanol for disinfection. From the abdominal region towards the dorsal region, a 4- to 5-cm incision was made in the gluteal region to expose the sciatic nerve. The epineurium and perineurium of the sciatic nerve were stripped off 1.5 cm, and further the surrounding muscle tissue was burned. Subsequently, the hydrogel of Example 4 (0.5 mL) was applied around the sciatic nerve having the epineurium and perineurium stripped off, and the muscle layer and skin at the incision site were sutured. The wound site was disinfected with an Isodine disinfectant, and the rat was then returned to the cage. In a week after surgery, the animals were subjected to sciatic nerve collection under pentobarbital sodium anesthesia, and Masson's trichrome staining was performed for histological observation of the sciatic nerve. As a result, regeneration of the perineurium was found in a week after surgery.

### [Comparative Example 3]

As control, the same operation as in Example 5 was performed without applying the hydrogel, and the sciatic nerve was histologically observed. As a result, the regeneration of the perineurium in a week after surgery was insufficient.

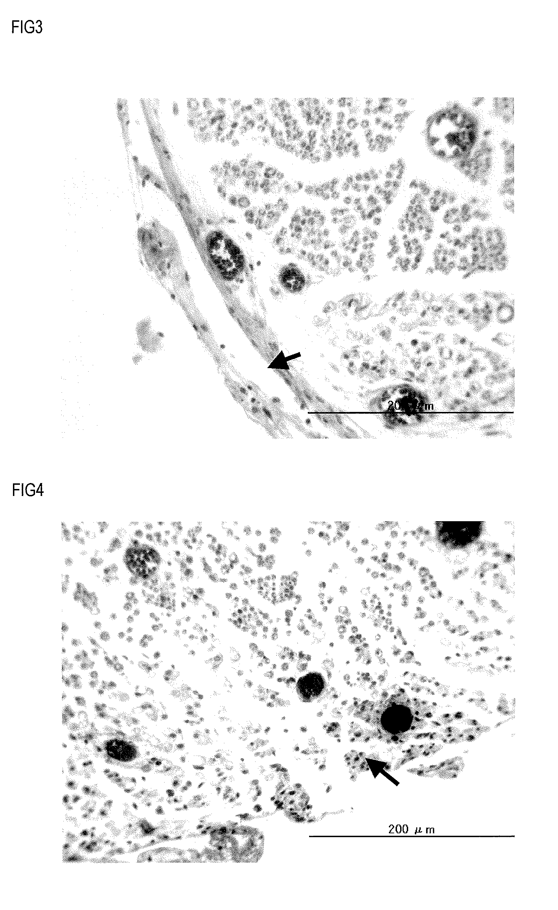

The results of Masson's trichrome staining in a week after surgery in Example 5 and Comparative Example 3 are shown in Figs. 3 and 4, respectively. As above, from the histological observations in a week after surgery, better regeneration of the perineurium was found in Example 5 than in Comparative Example 3. Therefore, it was confirmed that the hydrogel obtained in Example 4 is highly effective in restoring damaged or degenerated nerves in vivo.

### [Example 6]

### (Production of Rat Sciatic Nerve Degeneration)

Using Lewis rats (three rats) from Charles River Laboratories Japan, the sciatic nerve was degenerated in accordance with the method of Ohsumi et al. , [Hidehiko Ohsumi, Hitoshi Hirata, Takeshi Nagakura, Masaya Tsujii, Toshiko Sugimoto, Keiichi Miyamoto, Takeshi Horiuchi: Plastic and Reconstructive Surgery 116 (3) : 823-30, 2005]. That is, a rat was fixed in the lateral position under anesthesia with intraperitoneally administered pentobarbital sodium, and the gluteal region was shaved and then disinfected with ethanol for disinfection. From the abdominal region towards the dorsal region, a 4- to 5-cm incision was made in the gluteal region to expose the sciatic nerve. The epineurium and perineurium of the sciatic nerve was stripped off 1.5 cm, and further the surrounding muscle tissue was burned. Subsequently, the hydrogel of Example 4 (0.5 mL) was applied around the sciatic nerve having the epineurium and perineurium stripped off, and the muscle layer and skin at the incision site were sutured. The wound site was disinfected with an Isodine disinfectant, and the rat was then returned to the cage. In 6 weeks after surgery, the animals were subjected to sciatic nerve collection under pentobarbital sodium anesthesia, and toluidine blue staining was performed for histological observation of the sciatic nerve. As a result, regeneration of the myelin sheath was found in 6 weeks after surgery.

### [Comparative Example 4]

As control, the same operation as in Example 6 was performed without applying the hydrogel, and the sciatic nerve was histologically observed. As a result, the regeneration of the myelin sheath in 6 weeks after surgery was insufficient.

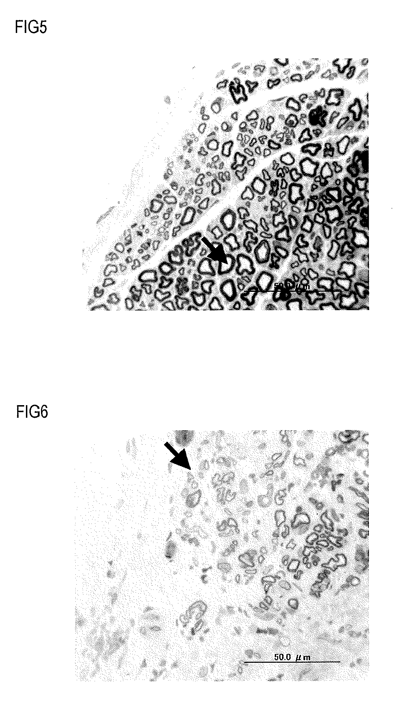

The results of toluidine blue staining in 6 weeks after surgery in Example 6 and Comparative Example 4 are shown in Figs. 5 and 6, respectively. As above, from the histological observations in 6 weeks after surgery, better regeneration of the myelin sheath was found in Example 6 than in Comparative Example 4. Therefore, it was confirmed that the hydrogel obtained in Example 4 is highly effective in restoring damaged or degenerated nerves in vivo.

### Industrial Applicability

The nerve dysfunction repairing material of the invention is a medical material that is injectable through a syringe and has excellent retention in the body, and is used for the surgical operation of a human, for example.

## Claims

1. A nerve dysfunction repairing material comprising a hydrogel of a polysaccharide derivative that has, in a 0.5 wt% aqueous solution, a complex modulus of 1 to 1000 N/m² and a loss factor of 0.01 to 2.0 as measured at an angular velocity of 10 rad/sec using a dynamic viscoelasticity measuring apparatus.

2. A nerve dysfunction repairing material according to claim 1, wherein the polysaccharide derivative is a cellulose derivative.

3. A nerve dysfunction repairing material according to claim 1, wherein the polysaccharide derivative is a cellulose derivative having a repeating unit represented by the following formula: 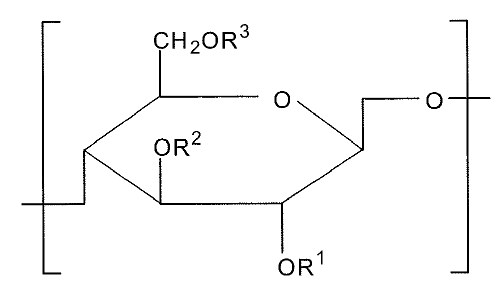 wherein R¹, R², and R³ are each independently selected from the group consisting of the following formulae (a), (b), (c), and (d) :
-H (a) ;
-CH₂-COOH (b) ;
-CH₂-COOX (c) ;
and 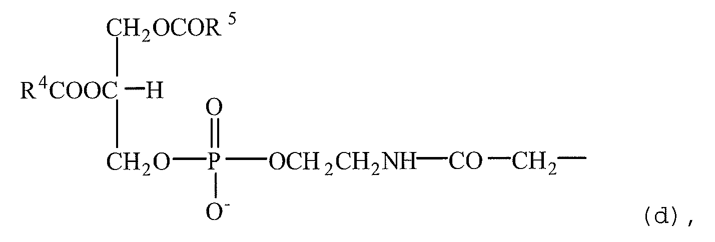 wherein
X in the formula (c) is an alkali metal or an alkaline-earth metal,
R⁴ and R⁵ in the formula (d) are each independently a C₉₋₂₇ alkyl group or alkenyl group,
the total degree of substitution of (b) and (c) is 0.3 to 2.0, and
the degree of substitution of (d) is 0.001 to 0.05.

4. A nerve dysfunction repairing material according to claim 3, wherein R⁴ and R⁵ are C₉₋₁₉ alkenyl groups.

5. A nerve dysfunction repairing material according to claim 3, wherein R⁴CO- and/or R⁵CO- is an oleoyl group.

6. A nerve dysfunction repairing material according to any one of claims 1 to 5, containing 0.1 to 1.5 parts by weight of the polysaccharide derivative per 100 parts by weight of water.

7. A nerve dysfunction repairing material comprising a hydrogel of a polysaccharide derivative, **characterized in that**, at a physiological salt concentration, the complex modulus thereof in a 1.0 wt% aqueous solution increases by 1 to 1000 N/m² as measured at an angular velocity of 10 rad/sec using a dynamic viscoelasticity measuring apparatus.

8. A nerve dysfunction repairing material according to claim 7, wherein the polysaccharide derivative is a cellulose derivative having a repeating unit represented by the following formula, and the complex modulus thereof in a 1.0 wt% aqueous solution is 5 to 200 N/m² as measured at an angular velocity of 10 rad/sec using a dynamic viscoelasticity measuring apparatus: 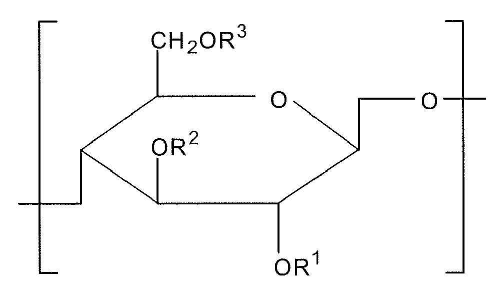 wherein R¹, R², and R³ are each independently selected from the group consisting of the following formulae (a), (b), (c), and (d):
-H (a) ;
-CH₂-COOH (b) ;
-CH₂-COOX (c) ;
and 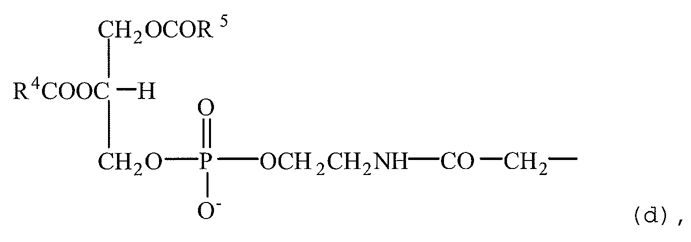 wherein
X in the formula (c) is an alkali metal or an alkaline-earth metal,
R⁴ and R⁵ in the formula (d) are each independently a C₉₋₂₇ alkyl group or alkenyl group,
the total degree of substitution of (b) and (c) is 0.3 to 2.0, and
the degree of substitution of (d) is 0.001 to 0.05.

9. A nerve dysfunction repairing material according to any one of claims 1 to 8, being a peripheral nerve dysfunction repairing material.

10. A nerve dysfunction repairing material according to claim 9, wherein the peripheral nerve dysfunction is an entrapment syndrome.

11. A nerve dysfunction repairing material according to any one of claims 1 to 8, being a central nerve dysfunction repairing material.

12. A nerve dysfunction repairing material according to any one of claims 1 to 8, being a sciatic nerve dysfunction repairing material.

13. A nerve dysfunction repairing material according to any one of claims 1 to 8, having a nerve conduction velocity improving effect.

14. A nerve dysfunction repairing material according to any one of claims 1 to 8, having a regenerative effect on the perineurium.

15. A nerve dysfunction repairing material according to any one of claims 1 to 8, having a regenerative effect on the myelin sheath.
